(19) Europäisches Patentamt
European Patent Office
Office européen des brevets

(11) Veröffentlichungsnummer: **0 166 693**
**B1**

(12) # EUROPÄISCHE PATENTSCHRIFT

(45) Veröffentlichungstag der Patentschrift: **11.07.90**

(51) Int. Cl.[5]: **C 07 D 209/76,** C 08 F 12/32

(21) Anmeldenummer: **85810288.2**

(22) Anmeldetag: **21.06.85**

(54) **Substituierte ungesättigte, hydroxylgruppenhaltige bicyclische Imide und deren Polymere.**

(30) Priorität: **27.06.84 CH 3095/84**

(43) Veröffentlichungstag der Anmeldung:
**02.01.86 Patentblatt 86/01**

(45) Bekanntmachung des Hinweises auf die Patenterteilung:
**11.07.90 Patentblatt 90/28**

(84) Benannte Vertragsstaaten:
**CH DE FR GB IT LI NL**

(56) Entgegenhaltungen:
**EP-A-0 105 024**
**US-A-3 450 711**

(73) Patentinhaber: **CIBA-GEIGY AG**
**Klybeckstrasse 141**
**CH-4002 Basel (CH)**

(72) Erfinder: **Renner, Alfred, Dr.**
**Marcoup 2**
**CH-3280 Muntelier (CH)**

**EP 0 166 693 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

**Beschreibung**

Die Erfindung betrifft mist Allyl oder Methallyl und gegebenenfalls Methyl substituierte hydroxylgruppenhaltige Bicyclo[2.2.1]hept-5-en-2,3-dicarbonsäureimide, deren Herstellung sowie die daraus gegebenenfalls in Anwesenheit anderer Comonomeren erhältlichen Polymeren.

Maleinimide und Bismaleinimide sowie N-Allyl-monomaleinimide sind bekannt.

Das US Patent 3,450,711 betrifft Bisimidverbindungen, hergestellt durch Umsetzung von Endo, cis-bicyclo[2.2.1]hept-5-en-2,3-dicarbonsäureanhydrid (= 5-Norbornen-2,3-dicarbonsäureanhydrid) mit ausgewählten organischen Diaminen. Diese Bisimide enthalten weder Methyl- noch Methallyl-oder Allylsubstituenten im Imidrest und unterschieden sich von den vorliegenden Verbindungen sowohl durch ihre Struktur als auch durch ihre chemische Reaktivität. Die Verbindungen gemäss diesem US Patent werden als Zwischenprodukte bei der Herstellung von Epoxidverbindungen verwendet.

Die EP-Patentanmeldung 0 105 024 beschreibt allyl- oder methallyl-substituierte Bicyclo[2.2.1]hept-5-en-2,3-dicarbonsäureimide und die daraus erhältlichen Polymeren.

Die erfindungsgemässen hydroxylgruppenhaltigen substituierten Bicyclo[2.2.1]hept-5-en-2,3-dicarbonsäureimide sind wertvolle Ausgangsprodukte, sowohl für Polymere mit ausgezeichneten Eigenschaften, als auch für von ihnen abgeleitete Sulfonsäureester. Sie sind durch die folgende Formel I gekennzeichnet:

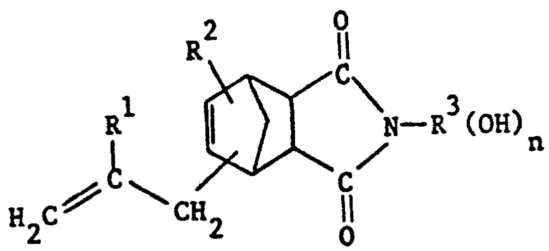

worin $R^1$ und $R^2$ unabhängig voneinander Wasserstoff oder Methyl und n 1, 2 oder 3 bedeuten, und $R^3$ eine direkte Bindung oder ein $C_2$—$C_{20}$-aliphatischer, ein ein- oder mehrkerniger $C_5$—$C_{20}$-cycloaliphatischer oder ein $C_6$—$C_{20}$-aromatischer Rest ist, für eine Gruppe der Formel II steht

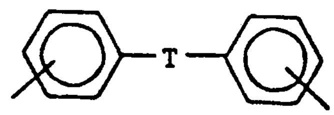

(II),

worin T Methylen, Isopropyliden, CO, O, S oder $20_2$ bedeutet, oder ein durch O-Atome in der Kette unterbrochener $C_2$—$C_{20}$-aliphatischer Rest der Formeln $—CH_2CH_2{+}OCH_2CH_2{]_m}$ oder $—CH_2CH_2CH_2{+}OCH_2CH_2CH_2{]_m}$ mit m = 1—10 ist, wobei die OH-Gruppen an verschiedene C-Atome von $R^3$ gebunden sind, und die Allyl- bzw. Methallylgruppe in 1- oder 6-Stellung des bicyclischen systems gebunden ist.

Vorzugsweise bedeuten $R^1$ und $R^2$ je ein Wasserstoffatom.

$R^3$ kann ein zwei- bis vierwertiger geradkettiger oder verzweigter aliphatischer Rest mit 2—20, vorzugsweise 2—12 und insbesondere 2—6 C-Atomen sein, der in der Kette durch ein oder mehrere Sauerstoffatome unterbrochen sein kann. Beispiele für geeignete aliphatische Rest $R^3$ sind Ethylen, 1,2- und 1,3-Propylen, Butylen, Penta- und Hexamethylen, Heptylen, Octylen, Decylen, Dodecylen, Hexadecylen, Neopentylen sowie Reste von Glycerin, von 1,1,1-Tris(hydroxymethyl)propan und von Pentaerythrit[2,2-Bis(hydroxymethyl)-1,3-propandiol]. Durch Sauerstoffatome unterbrochene aliphatische Rest werden von Ethylenglykol oder Propylenglykol abgeleitet und entsprechen Gruppen der Formeln $—CH_2CH_2{+}OCH_2CH_2{]_m}$ oder $—CH_2CH_2CH_2{+}OCH_2CH_2CH_2{]_m}$ mit m = 1—10.

$R^3$ kann auch ein ein- oder mehrkerniger cycloaliphatischer, insbesondere zweiwertiger Rest mit 5—20 C-Atomen sein, wie beispielsweise Cyclopentylen, Cyclohexylen, Cycloheptylen, Cyclooctylen, Bis(cyclohexylen)methan, 2,2-Bis(cyclohexylen)propan und Decalinylen.

Wenn $R^3$ einen aromatischen Rest bedeutet, so handelt es sich vorzugsweise um zweiwertige Reste, wie z.B. 1,3- oder 1,4-Phenylen oder Naphthylen, die jeweils, falls gewünscht, auch durch eine oder mehrere $C_{1-4}$-Alkylgruppen, wie Methyl, Ethyl oder Propyl, substituiert sein können. Bevorzugt sind die genannten Gruppen unsubstituiert, 1,3- und 1,4-Phenylengruppen sind als aromatische Reste besonders bevorzugt.

Wenn $R^3$ eine Gruppe der Formel II bedeutet, steht T vorzugsweise für O, $SO_2$ und insbesondere für Methylen oder Isopropyliden.

Besonders bevorzugte Verbindungen der Formel I sind solche, worin $R^3$ eine direkte Bindung, Gruppen

$—C_rH_{2r}—$ mit r = 2—6, $—CH_2CH_2—[OCH_2CH_2]_m—$ mit m = 2 und insbesondere 1, einen Rest von Glycerin, einen einkernigen zweiwertigen cycloaliphatischen Rest mit 5—8 C-Atomen, wie Cyclopentylen und Cyclohexylen, einen unsubstituierten zweiwertigen $C_6—C_{10}$ aromatischen Rest, wie 1,3- und 1,4-Phenylen oder Naphthylen oder eine Gruppe der Formel II bedeutet, wobei T für O, $SO_2$ und insbesondere für Methylen oder Isopropyliden steht.

Ganz besonders bevorzugt sind Verbindungen der Formel I, worin $R^3$ eine direkte Bindung, den Rest

$-CH_2CH_2-$, 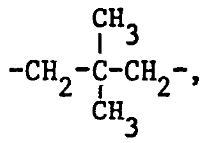, $-CH_2CH_2OCH_2CH_2-$, 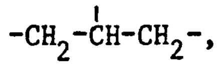,

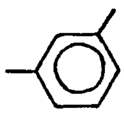, 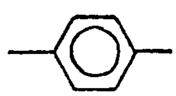 oder 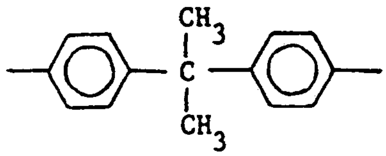

bedeutet.

Am meisten bevorzugt sind Verbindungen der Formel I, worin $R^3$ eine direkte Bindung, Ethylen oder 1,4-Phenylen bedeutet.

Die erfindungsgemässen Imide können auf an sich bekannte Weise z.B. durch Umsetzen eines Anhydrids der Formel III

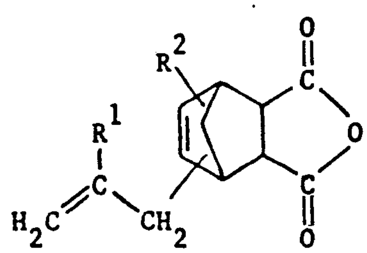 (III)

mit einer Verbindung der Formel IV

$$H_2N—R^3(OH)_n \qquad (IV),$$

worin $R^1$, $R^2$, $R^3$ und n die unter Formel I angegebene Bedeutung haben, bei erhöhter Temperatur und unter Abdestillieren des bei der Reaktion entstehenden Wassers hergestellt werden. Sofern es sich bei den Verbindungen der Formel IV um niedrigsiedende Verbindungen handelt, ist ein Ueberschuss dieser Reaktanden zu empfehlen. Die Umsetzung kann ohne Lösungsmittel oder in Gegenwart eines inerten Lösungsmittels, das für die azeotrope Entfernung des Wassers verwendbar ist (Schleppmittel) erfolgen. Die Temperatur der Umsetzung kann zwischen 100 und 250°C liegen. Bevorzugt werden die Imide der Formel I in der Schmelze bei einem Druck von höchstens 4500 Pa bei Temperaturen zwischen 130 und 220°C, insbesondere 180 und 220°C, hergestellt.

Ueberraschenderweise reagieren Verbindungen der Formel IV ebenso gut mit Anhydriden der Formel III wie OH-freie Amine und führen nicht zu durch Reaktionen der Hydroxylgruppe verursachten Produktegemischen. Die Ausgangsstoffe der Formel III können gemäss dem in der US-Patentschrift 3,105,839 beschriebenen Verfahren hergestellt werden, indem man Natrium-Cyclopentadienid oder Natrium-methylcyclopentadienid mit einem Allyl- oder Methallylhalogenid umsetzt, worauf sich eine Diels-Alder-Reaktion mit Maleinsäureanhydrid anschliesst. Obgleich in der US Patentschrift angegeben wird, dass die Allylgruppe in 7-Stellung bis bicyclischen Systems gebunden ist, zeigen neuere Untersuchungen, dass eine isomere Mischung in Bezug auf die Stellung der Allylgruppe (in 1 und 6 Position) und auch auf die Endo- und Exokonfiguration des Anhydridteils gebildet wird. Die isomeren Komponenten konnten bisher nur durch präparative Gaschromatographie isoliert werden.

Verbindungen der Formel IV sind bekannt oder können nach an sich bekannten Verfahren hergestellt werden.

Die erfindungsgemässen Verbindungen sind flüssige oder niedrigschmelzende feste Stoffe, die zu festen Produkten mit hoher Glasumwandlungstemperatur polymerisiert werden können.

Dank der Anwesenheit von zwei verschiedenartigen reaktiven Gruppen, der Doppelbindungen sowie der Hydroxylgruppen, können die erfindungsgemässen Imide als Edukte bzw. Zwischenprodukte für die Herstellung verschiedenartiger Polymeren verwendet werden. So können die Imide der Formel I z.B. mit

Carboxylendgruppen aufweisenden Polyestern, mit Polyisocyanaten oder Polybutadienen umgesetzt werden.

Die Imide der Formel I können auch als Monomere oder Comonomere zur Herstellung von neuen Polymeren verwendet werden. Die Umsetzung kann z.B. durch Erhitzen während 6—60 Stunden auf eine Temperatur zwischen 180 und 300°C, vorzugsweise zwischen 200 und 250°C durchgeführt werden. Dabei gilt in Bezug auf bevorzugte Bedeutungen von $R^1$, $R^2$, $R^3$ und n das oben Angegebene.

Die erfindungsgemässen Verbindungen können direkt verwendet und polymerisiert bzw. kondensiert werden, oder sie können zunächst in enem organischen Lösungsmittel, wie Toluol, Xylol, Methylethylketon, Ethylenglykolmonoalkyl- und -dialkylethern mit 1—4 C-Atomen in den Alkylgruppen oder einen ähnlichen, in der Lackindustrie üblichen Lösungsmittel, gelöst werden. Solche Lösungen können als Imprägniermittel oder Beschichtungsmittel oder auch als Versandobjekt an den Verbraucher dienen.

Ein bevorzugtes Anwendungsgebiet der erfindungsgemässen Imide ist deren Umsetzung mit Epoxidharzen, wobei gehärtete Produkte mit ausgezeichneten Eigenschaften erhalten werden.

Gegenstand der vorliegenden Anmeldung sind somit auch härtbare Gemische enthaltend Imide der Formel I, Epoxidharze und gegebenenfalls weitere übliche Zusätze, wie z.B. Katalysatoren (Härtungsbeschleuniger). Ein weiterer Gegenstand der Erfindung sind Polymere, die dadurch erhältlich sind, dass man Imide der Formel I mit Epoxidharzen, vorzugsweise in Anwesenheit eines Katalysators, umsetzt. Dabei werden als Imide vorzugsweise erfindungsgemässe N-Hydroxyarl-Derivate verwendet.

Die zu verwendenden Epoxidharze haben vorzugsweise im Durchschnitt mehr als eine Epoxidgruppe pro Molekül. Insbesondere seien genannt:

Alicyclische Polyepoxide, wie Epoxyethyl-3,4-epoxycyclohexan (Vinylcyclohexendiepoxid), Limonendiepoxid, Dicyclopentadiendiepoxid, Bis(3,4-epoxycyclohexylmethyl)adipat, 3',4'-Epoxycyclohexylmethyl-3,4-epoxycyclohexancarboxylat, 3',4'-Epoxy-6'-methylcyclohexylmethyl-3,4-epoxy-6-methylcyclohexancarboxylat, 3-(3',4'-Epoxycyclohexyl)-2,4-dioxaspiro[5,5]-8,9-epoxyundecan, 3-Glycidyloxyethoxyethyl-2,4-dioxaspiro[5,5]-8,9-epoxyundecan.

Di- oder Polyglycidylether von mehrwertigen Alkoholen, wie, 1,4-Butandiol oder Polyalkylenglykolen, wie Polypropylenglykole, Di- oder Polyglycidylether von cycloaliphatischen Polyolen, wie 2,2-Bis(4'-hydroxycyclohexyl)propan, Di- oder Polyglycidylether von mehrwertigen Phenolen, wie Resorcin, Bis(4-hyroxyphenyl)methan (Bisphenol F), 2,2-Bis(4'-hydroxyphenyl)propan (Bisphenol A), 2,2-Bis(4'-hydroxy-3',5'-dibromphenyl)propan, 1,1,2,2-Tetrakis(4'-hydroxyphenyl)ethan, oder unter saueren Bedingungen erhaltene Kondensationsprodukte von Phenolen mit Formaldehyd, wie Phenol-Novolake und Kresol-Novolake; ferner Di- oder Poly(β-methylglycidyl)ether der oben angeführten Polyalkohole und Polyphenole.

Polyglycidylester und Poly(β-methylglycidyl)ester von mehrwertigen Carbonsäuren, wie Phthalsäure, Terephthalsäure, Tetrahydrophthalsäure und Hexahydrophthalsäure.

N-Glycidylderivate von Aminen, Amiden und heterocyclischen Stickstoffbasen, wie N,N-Diglycidylanilin, N,N-Diglycidyltoluidin, N,N,N',N'-Tetraglycidyl-bis(4-aminophenyl)methan, Triglycidylisocyanurat, N,N'-Diglycidylethylharnstoff, N,N'-Diglycidyl-5,5-dimethylhydantoin, N,N'-Diglycidyl-5-isopropylhydantoin, N,N'-Diglycidyl-5,5-dimethyl-6-isopropyl-5,6-diyhdrouracil.

Besonders bevorzugt sind Polyglycidylether von Phenol- oder Kresol-Formaldehyd Novolaken sowie Diglycidylether von Bisphenol A und Bisphenol F.

Geeignete Katalysatoren (Beschleuniger) sind z.B. tertiäre Amine, deren Salze oder quarternäre Ammoniumverbindungen, z.B. Benzyldimethylmain, 2,4,6-Tris(dimethylaminomethyl)phenol, 1-Methylimidazol, 2-Ethyl-4-methylimidazol, 2-Phenylimidazol, 4-Aminopyridin, Tripentylammoniumphenolat; oder Alkalimetallalkoholate, wie z.B. Natriumhexantriolat. Die Umsetzung (Härtung) der erfinungsgemässen Mischungen wird zweckmässig im Temperaturintervall von 50°C bis 300°C, bevorzugt von 150—300°C, durchgeführt.

Der bevorzugte Härtungsbeschleuniger ist 2-Phenylimidazol.

Man kann die Härtung in bekannter Weise auch zwei- oder mehrstufig durchführen, wobei die erste Härtungsstufe bei niedriger Temperatur und die Nachhärtung bei höherer Temperatur durchgeführt wird.

Die Härtung kann gewünschtenfalls auch derart in 2 Stufen erfolgen, dass die Härtungsreaktion zunächst vorzeitig abgebrochen bzw. die erste Stufe bei wenig erhöhter Temperatur durchgeführt wird, wobei ein noch schmelzbares und/oder lösliches, härtbares Vorkondensat (sogenannte "B-Stufe") aus der Imid-Komponente (a) und der Epoxid-Komponente (b) erhalten wird. Ein derartiges Vorkondensat kann z.B. zur Herstellung von "Prepregs", Pressmassen oder Sinterpulvern dienen.

Der Ausdruck "Härten", wie er hier gebraucht wird, bedeutet die Umwandlung der löslichen, entweder flüssigen oder schmelzbaren Epoxidharze in feste, unlösliche und unschmelzbare, dreidimensional vernetzte Produkte bzw. Werkstoffe, und zwar in der Regel unter gleichzeitiger Formgebung zu Formkörpern, wie Giesskörpern, Presskörpern und Schichtstoffen, zu Imprägnierungen, Beschichtungen, Lackfilmen oder Verklebungen.

Selbstverständlich können den Imiden der Formel I inerte und stabile Stoffe, wie Füllmittel, Pigmente, Farbstoffe und andere Additive, zugegeben werden, bevor sie zu vernetzten Gebilden polymerisiert werden.

Die erfindungsgemässen Imide der Formel (I), insbesondere solche mit n = 1, können z.B. mit einem Sulfonsäurechlorid der Formel

$$R^4—SO_2Cl \qquad (V)$$

unter Kühlung in Gegenwart eines HCl-Akzeptors zu den entsprechenden Sulfonsäureestern umgesetzt werden, wobei $R^4$ $C_1$—$C_6$-Alkyl, $C_5$—$C_6$-Cycloalkyl, $C_6$—$C_{10}$-Aryl oder $C_7$—$C_{12}$-Alkaryl, und vorzugsweise Methyl Phenyl oder p-Tolyl bedeutet.

Die Umsetzung von Verbindungen der Formel I mit den Sulfonsäurechloriden der Formel V erfolgt vorzugsweise mit einem äquimolaren Verhältnis der Reaktanden in einem inerten Lösungsmittel mit einem Siedepunkt unter 200°C. Als Lösungsmittel kommen beispielsweise aliphatische oder alicyclische, gegebenenfalls chlorierte Kohlenwasserstoffe und insbesondere aromatische, gegebenenfalls chlorierte Kohlenwassserstoffe, wie z.B. Chlorbenzol, Xylol und insbesondere Toluol in Betracht. Um die während der Reaktion entstehende Salzsäure zu binden, wird die Umsetzung in Gegenwart eines HCl-Akzeptors, vorzugsweise eines tertiären Amins, wie z.B. Triethylamin, Dimethylanilin, Pyridin oder Lutidin, durchgeführt. Da die Reaktion exotherm verläuft, wird mit Kühlung gearbeitet, so dass die Temperatur des Reaktionsgemisches vorzugsweise 10°C nicht übersteigt.

Die auf diese Art erhältlichen Verbindungen sind flüssige oder niedrigschmelzende feste Stoffe. Bei erhöhter Temperatur setzen sie die entsprechende Sulfonsäure, $R^4SO_3H$, frei, wobei $R^4$ die oben angegebene Bedeutung hat, und eignen sich dadurch als latente Katalysatoren für die Vernetzung von kationisch polymerisierbarem Material. Der nach der Abspaltung der Sulfonsäure verbliebene Rest der Verbindungen wird dank seiner Polyfunkitionalität in das Endpolymere mit eingebaut. Es kann auch ein Gemisch von mehreren von oben beschriebenen Verbindungen als Katalysator eingesetzt werden.

Geeignete kationisch polymerisierbare Materialien, für welche die oben erwähnten Sulfonsäureester als Katalysatoren eingesetzt werden können, sind u.a. substituierte ungesättigte bicyclische Imide wie sie Z.B. in der EP-Patentanmeldung 0 105 024 beschrieben sind. Die so erhatlenen vernetzten Polymere zeichnen sich durch vorzügliche mechanische und thermische Eigenschaften aus.

In den folgenden Beispielen werden die Herstellung einiger erfindungsgemässer Imide, deren Eigenschaften und Anwendung illustriert.

## Herstellungsbeispiele

### Beispiel 1

Methallylbicyclo[2.2.1]hept-5-en-2,3-dicarbonsäure-N-hydroxylimid

Man löst 34,75 g Hydroxylamin-hydrochlorid in Wasser und setzt 109 g Methallylbicyclo[2.2.1]hept-5-en-2,3-dicarbonsäureanhydrid zu. Man tropft unter Rühren 40 g 50%ige wässrige Natronlauge zu, erhitzt 1 h auf Rückfluss, destilliert danach alle flüchtigen Anteile ab, nimmt den Rückstand in Toluol auf, filtriert vom ausgeschidenen Natriumchlorid ab und entfernt das Toluol am Rotationsverdampfer (150°C, 2000 Pa). Es werden 98,5 einer hochviskosen Flüssigkeit (90% der Theorie) erhalten, die bis 200°C/2,5 Pa nicht destillierbar ist, da das Produkt vermutlich als Dimeres vorliegt.

| Analyse $C_{13}H_{13}NO_2$ | ber. | gef. |
|---|---|---|
| % C | 71,23 | 71,08 |
| % H | 5,98 | 6,07 |
| % N | 6,39 | 6,51 |

### Beispiel 2

Allylbicyclo[2.2.1]hept-5-en-2,3-dicarbonsäure-N-(2'-hydroxyethyl)imid

Man legt 760 g Allylbicyclo[2.2.1]hept-5-en-2,3-dicarbonsäureanhydrid (Isomerengemisch) vor und tropft unter Rühren 227,55 g Monoethanolamin ein. Man erhitzt während 2 Stunden auf Rückfluss und destilliert sodann Wasser und etwas überschüssiges Monoethanolamin ab, bis eine Temperatur von 175°C erreicht ist. Danach erniedrigt man den Druck auf 2670 Pa. Man kühlt auf 100°C ab und rektifiziert das Produkt bei 4,9 Pa. Zwischen 170 und 174°C gehen 712,4 g (77,3% d.Th.) eines blassgelben Oels mit folgenden Kenndaten über:

$$n_{25}^{D} = 1{,}5344$$

$$\eta_{25} = 2{,}43 \text{ Pa.s}$$

| Analyse: | ber. | gef. |
|---|---|---|
| % C | 68,00 | 68,08 |
| % H | 6,93 | 7,06 |
| % N | 5,66 | 5,61 |

IR-Spektrum:
1619 $cm^{-1}$ cyclische Doppelbindung
1641 $cm^{-1}$ Allylgruppe
1697 $cm^{-1}$ Carbonylgruppe
1768 $cm^{-1}$ Carbonyl im cyclischen Imid
3449 $cm^{-1}$ Hydroxylgruppe

Das Verfahrensprodukt ist wie das Ausgangsmaterial ein Gemisch aus dem 1 exo-, 6 exo-, 1 endo- und 6 endo-Isomeren.

Beispiel 3

Allylbicyclo[2.2.1]hept-5-en-2,3-dicarbonsäure-N-(2',2'-dimethyl-3'-hydroxypropyl)imid

Man setzt 204 g Allylbicyclo[2.2.1]hept-5-en-2,3-dicarbonsäureanhydrid (Isomerengemisch) mit 103 g Neopentanolamin während 4 Stunden bei 120°C um, und destilliert das Produkt. Man erhält zwischen 169 und 172°C bei 2,53 Pa 268 g (92,7% d.Th.) eines gelben Oels mit einer Brechungszahl $n_{25}^{D}$ von 1,5190 und einer Viskosität von 6,21 Pa.s.

| Analyse: | ber. | gef. |
|---|---|---|
| % C | 70,56 | 70,67 |
| % H | 8,01 | 8,13 |
| % N | 4,84 | 4,77 |
| % OH | 5,88 | 5,69 |

Der Hydroxylgruppengehalt wird durch Acetylierung mit Essigsäureeanhydrid in Pyridin bestimmt.

Beispiel 4

Allylbicyclo[2.2.1]hept-5-en-2,3-dicarbonsäure-N-[2'-(2''-hydroxyethoxy)ethyl]imid

Man setzt 120 g Allylbicyclo[2.2.1]hept-5-en-2,3-dicarbonsäureanhydrid (Isomerengemisch) mit 67,9 g Diglykolamin bei 140°C um, erhitzt auf 200°C und erniedrigt den Druck auf 10,6 Pa. Man erhält 142,6 g eines gelben Oels mit einer Viskosität von 1,55 Pa.s bei 25°C und einer $n_{25}^{D}$ von 1,5230.

| Analyse: | ber. | gef. |
|---|---|---|
| % C | 65,96 | 65,74 |
| % H | 7,27 | 7,39 |
| % N | 4,81 | 4,71 |
| % OH | 5,84 | 5,34 |

Beispiel 5

Allylbicyclo[2.2.1]-hept-5-en-2,3-dicarbonsäure-N-(2',3'-dihydroxypropyl)imid

Man erhitzt 102 g Allybicyclo[2.2.1]hept-5-en-2,3-dicarbonsäurehydrid mit 45,55 g 2,3-Dihydroxypropylamin auf 200°C und erniedrigt den Druck auf 47 Pa. Man erhält 138 g (96,5% d.Th.) eines hochviskosen Produkts mit $\eta_{80}$ = 605 mPa.s und $n_{25}^{D}$ = 1,5380.

| Analyse: | ber. | gef. |
|---|---|---|
| % C | 64,97 | 64,48 |
| % H | 6,91 | 7,02 |
| % N | 5,05 | 4,96 |
| % OH | 6,13 | 5,95 |

Beispiel 6

Allylbicyclo[2.2.1]hept-5-en-2,3-dicarbonsäure-N-(4'-hydroxyphenyl)imid

Man erhitzt 408 g eines Isomerengemisches von Allylbicyclo[2.2.1]hept-5-en-2,3-dicarbonsäureanhydrid mit 238,26 g 4-Aminophenol auf 200°C, erniedrigt den Druck auf 2,7 Pa und hält 1 Stunde unter diesen Bedingungen. Man erhält 535 g eines roten Festharzes (87,7% d.Th.) mit einer Glasumwandlungstemperatur von 58°C.

| Analyse: | ber. | gef. |
|---|---|---|
| % C | 73,20 | 72,43 |
| % H | 5,90 | 6,05 |
| % N | 4,89 | 4,90 |
| % OH | 5,76 | 5,38 |

Beispiel 7

Allylbicyclo[2.2.1]hept-5-en-2,3-dicarbonsäure-N-(3'-hydroxyphenyl)imid

Man verfährt wie im vorhergehenden Beispiel 6, verwendet aber 3-Aminophenol. Man erhält ein rotbraunes Festharz mit einer Glasumwandlu harz mit einer Glasumwandlungstemperatur von 61°C, Ausbeute 97,5% der Theorie.

| Analyse: | ber. | gef. |
|---|---|---|
| % C | 73,20 | 72,56 |
| % H | 5,80 | 6,02 |
| % N | 4,74 | 5,97 |
| % OH | 5,76 | 5,66 |

Beispiel 8

Allylmethylbicyclo[2.2.1]hept-5-en-2,3-dicarbonsäure-N-[4'-(4''-hydroxyphenylisopropyliden)phenyl]imid

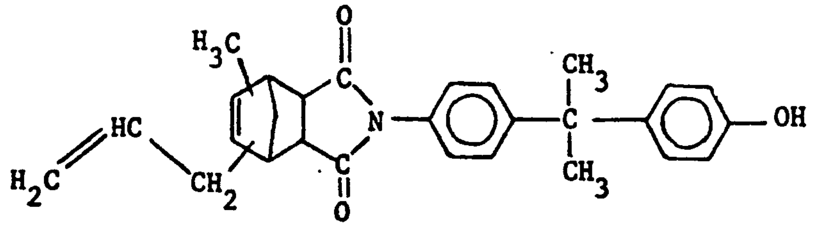

Man setzt 54 g Allylmethylbicyclo[2.2.1]hept-5-en-2,3-dicarbonsäureeanhydrid mit 56,74 g 2-(4'-Aminophenyl)-2-(4''-hydroxyphenyl)propan bei 200°C und 2,5 Pa um. Man erhält 100,5 g eines dunkelbraunen Festharzes (97,3% d.Th.) mit einer Glasumwandlungstemperatur von 100°C.

| Analyse: | ber. | gef. |
|---|---|---|
| % C | 78,45 | 78,15 |
| % H | 6,58 | 6,54 |
| % N | 3,39 | 3,16 |

Beipiel 9

Allylbicyclo[2.2.1]hept-5-en-2,3-dicarbonsäure-N-hydroxylimid

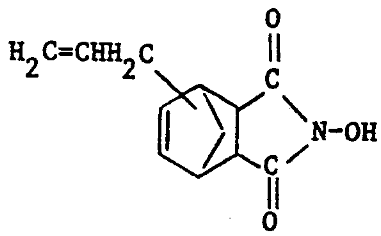

Man löst 139 g (2 mol) Hydroxylaminhydrochlorid in 200 ml Wasser, fügt 408 g (2 mol) Allylbicyclo-[2.2.1]hept-5-en-2,3-dicarbonsäureanhydrid (hergestellt gemäss Beispiel 1 der US-Patentschrift 3 105 839) zu und tropft unter kräftigem Rühren 160 g 50%ige wässrige Natronlauge ein. Danach erhitzt man 1 Stunde auf Rückfluss, destilliert das Wasser und Spuren öliger Bestandteile ab, nimmt den Rückstand in Toluol auf, filtriert, vom Kochsalz ab und entfernt das Toluol am Rotationsverdampfer bei 150°C und 2000 Pa. Es hinterbleiben 402,6 g des Produkts (91,5% d.Th.) als hellbraune viskose Flüssigkeit mit einer Viskosität von 1,28 Pa.s bei 80°C.

| Analyse: | ber. | gef. |
|---|---|---|
| % C | 65,74 | 65,34 |
| % H | 5,98 | 6.02 |
| % N | 6,39 | 6.40 |

Anwendungsbeispiele

Beispiele I—V

Man schmilzt jeweils die in der folgenden Tabelle angegebenen Imide und Epoxidverbindungen bei ca. 150°C zusammen, gibt 2-Phenylimidazol als Katalysator hinzu, entgast im Vakuum und giesst die Harzschmelzen in Plattenformen von 120 × 120 × 4 mm³. Man härtet während 3 h bei 200°C, 3 h bei 220°C und 12 h bei 250°C und erhält einwandfreie, dunkelrot gefärbte, zäh-harte Platten, die zu Prüfstäben zersägt werden. An diesen werden die in der Tabelle aufgeführten Eigenschaften gemessen.

Tabelle

| Beispiel Nr. | I | II | III | IV | V |
|---|---|---|---|---|---|
| Produkt aus Herstellungsbeispiel Nr. | 6 | 7 | 8 | 7 | 8 |
| Menge (g) | 60 | 60 | 60 | 60 | 60 |
| Polyglycidylierter Phenol-Formaldehyd Novolak[a)] (g) | 35,7 | 35,7 | - | - | - |
| Bisphenol A Diglycidylether[b)] (g) | - | - | 32,5 | 32,5 | 27,2 |
| 2-Phenylimidazol (mg) | 95,6 | 95,6 | 92,5 | 92,5 | 87,2 |
| Biegefestigkeit (ISO 178) ($N/mm^2$) | 118 | 70,9 | - | 85,2 | - |
| Nach 14 Tagen in Wasser bei 71°C ($N/mm^2$) | 42,2 | 56,4 | - | 48,6 | - |
| Randfaserdehnung (%) | 5,1 | 1,95 | - | 2,2 | - |
| Nach 14 Tagen in Wasser bei 71°C (%) | 1,5 | 1,59 | - | 1,5 | - |
| Schlagbiegefestigkeit (ISO 179) ($kJ/m^2$) | 23,2 | 4,3 | - | 6,3 | - |
| Nach 14 Tagen in Wasser bei 71°C ($kJ/m^2$) | 20,4 | 4,7 | - | 6,5 | - |
| Wasseraufnahme nach 14 Tagen bei 71°C (%) | 3,2 | 2,7 | - | 3,1 | - |
| Glasumwandlungstemperatur (°C) (Mettler TA 2000) | 222,5 | 193 | 204 | 188 | 186 |

a) Epoxidgehalt 5,7 Aequivalent/kg

b) Epoxidgehalt 5,3 Aequivalent/kg

Beispiel VI

a) Herstellung von Allylbicyclo[2.2.1]hept-5-en-2,3-dicarbonsäure-N-benzolsulfonyloxyimid

Man löst das gemäss Beispiel 9 erhaltene Produkt in 1 l Toluol und gibt 222,8 g Triethylamin zu, rührt bis eine homogene Lösung erhalten wird und kühlt auf 0°C. Man tropft unter starkem Rühren und unter äusserer Kühlung 324,7 g Benzolsulfonylchlorid so ein, dass die Temperatur des Reaktionsgemisches zwischen 5 und 10°C verbleibt. Man rührt über Nacht bei Raumtemperatur, versetzt mit Wasser, stellt mit konzentrierter Salzsäure auf pH = 5 ein und wäscht noch 2 mal mit Wasser von 75°C. Nach der Abtrennung der wässrigen Phasen trocknet man über $Na_2SO_4$, filtriert und dampft am Rotationsverdampfer bei 110°C und 2000 Pa ein. Man erhält 471 g einer viskosen Flüssigkeit, die beim Stehen kristallisiert (Fp. = 97,99°C).

| Elementaranalyse: | ber. | gef. |
|---|---|---|
| %C: | 60,16 | 60,35 |
| %H: | 4,77 | 4,85 |
| %N: | 3,90 | 3,90 |
| %S: | 8,92 | 8,59 |

IR-Spektrum ($cm^{-1}$): 575,4, 685,5 und 736,2 Aryl; 1195 und 1398 —$SO_2O$—; 1620 cycl. Doppelbindung: 1604 Allyldoppelbindung; 1742 Carbonyl.

b) Verwendung als Katalysator für die kationische Polymerisation.

Das unten angegebene Harz wird in Gegenwart des unter a) hergestellten Sulfonyloxy-Imids, welches als latenter Katalysator wirkt, gehärtet.

*Harz:* Bis[4-(allylbicyclo[2.2.1]hept-5-en-2,3-dicarbonsäureimidophenyl)methan], hergestellt gemäss Beispiel 11 der EP—A 0 105 024.

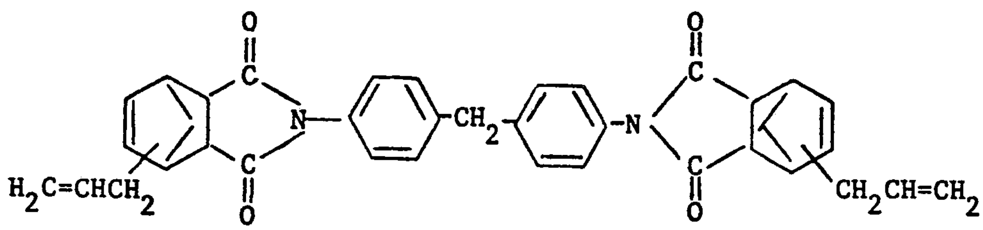

Das Harz wird mit 1% des Katalysators versetzt erhitzt, evakuiert, in Stahlformen von 150 × 150 × 4 $mm^3$ gegossen und während 2 Stunden bei 190°C und 2 Stunden bei 250°C gehärtet.

Das gehärtete Produkt hat die folgenden Eigenschaften:

| | |
|---|---|
| Biegefestigkeit (ISO 178) | 99,0 $N/mm^2$ |
| Randfaserdehnung (ISO 178) | 3,4% |
| Schlagbiegefestigkeit (ISO 179) | 6,1 $kJ/m^2$ |
| Glasumwandlungstemperatur (Mettler TA 2000) | 280°C. |

**Patentansprüche**

1. Imide der Formel

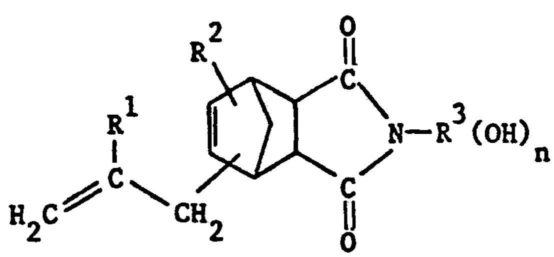

(I),

worin $R^1$ und $R^2$ unabhängig voneinander Wasserstoff oder Methyl und n 1, 2 oder 3 bedeuten, und $R^3$ eine direkte Bindung oder ein $C_2$—$C_{20}$-aliphatischer, ein ein- oder mehrkerniger $C_5$—$C_{20}$-cycloaliphatischer oder ein $C_6$—$C_{20}$-aromatischer Rest ist, für eine Gruppe der Formel II steht

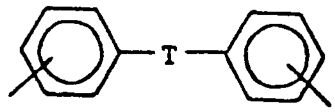

(II),

worin T Methylen, Isopropyliden, CO, O, S oder $SO_2$ bedeutet, oder ein durch O-Atome in der Kette unterbrochener $C_2$—$C_{20}$-aliphatischer Rest der Formeln $—CH_2CH_2—[OCH_2CH_2]_m—$ oder $—CH_2CH_2CH_2—[OCH_2CH_2CH_2]_m—$ mit m = 1—10 ist, wobei die OH-Gruppen an verschiedene C-Atome von $R^3$ gebunden sind, und die Allyl- bzw. Methallylgruppe in 1- oder 6-Stellung des bicyclischen systems gebunden ist.

2. Imide der Formel I nach Anspruch 1, worin $R^1$ und $R^2$ je Wasserstoff bedeuten.

3. Imide der Formel I nach Anspruch 1, worin $R^3$ eine direkte Bindung, eine Gruppe $—C_rH_{2r}—$ mit r = 2—6, eine Gruppe $—CH_2CH_2—[OCH_2CH_2]_m—$ mit m = 2 und inbesondere 1, einen rest von Glycerin, einen einkernigen zweiwertigen cycloaliphatischen rest mit 5—8 C-Atomen, einen unsubstituierten zweiwertigen $C_6$—$C_{10}$ aromatischen Rest oder eine Gruppe der Formel II bedeutet, wobei T für O, $SO_2$ und insbesondere für Methylen oder Isopropyliden steht.

4. Imide der Formel I nach Anspruch 1, worin $R^3$ eine direkte Bindung, den Rest.

$-CH_2CH_2-$, 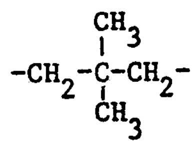, $-CH_2CH_2OCH_2CH_2-$, 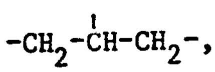,

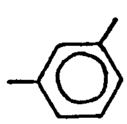 , 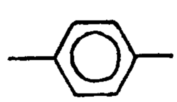 oder 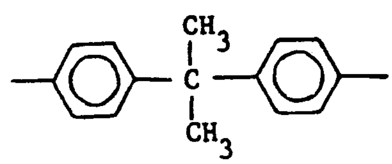

bedeutet.

5. Imide der Formel I nach Anspruch 1, worin $R^3$ eine direkte Bindung, Ethhylen oder 1,4-Phenylen bedeutet.

6. Verfahren zur Herstellung von Imiden der Formel I nach Anspruch 1, dadurch gekennzeichnet, dass man ein Anhydrid der Formel III

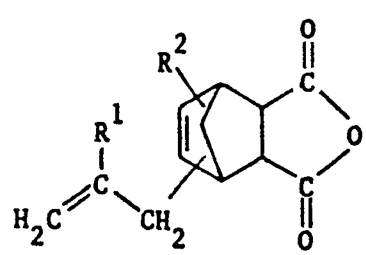

(III)

bei erhöhter Temperatur und unter Abdestillieren des bei der Reaktion entstehenden Wassers mit einer Verbindung der Formel IV

$$H_2N—R^3(OH)_n \quad (IV)$$

umsetzt, wobei $R^1$, $R^2$, $R^3$ und n die im Anspruch 1 angegebene Bedeutung haben.

7. Verwendung der Imide der Formel I nach Anspruch 1 zusammen mit einem oder mehreren Epoxidharzen und gegebenenfalls weiteren üblichen Zusätzen zur Herstellung gehärteter Produkte.

**Revendications**

1. Imides répondant à la formule:

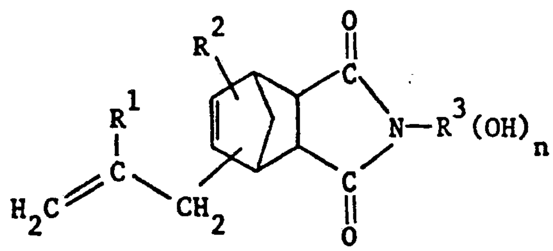

(I),

dans laquelle: $R^1$ et $R^2$ représentent chacun, indépendamment l'un de l'autre, l'hydrogène ou un méthyle, n désigne un nombre égal à 1, à 2 ou à 3 et $R_3$ représente une liaison directe, un radical aliphatique en $C_2$—$C_{20}$, un radical cycloaliphatique en $C_5$—$C_{20}$, à un ou plusieurs noyaux, un radical aromatique en $C_6$—$C_{20}$, à un ou plusieurs noyaux, un radical répondant à la formule II:

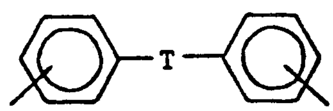

(II),

dans laquelle T représente un méthylène, un isopropylidène, CO, O, S ou $SO_3$, ou un radical aliphatique en $C_2$—$C_{20}$ dont la chaîne est interrompue par des atomes d'oxygène et qui répond à l'une des formules: $—CH_2CH_2{+}OCH_2CH_2]_m$ et $—CH_2CH_2CH_2{+}OCH_2CH_2]_m$ dans lesquelles m désigne un nombre de 1 à 10, les radicaux —OH étant portés par des atomes de carbone différents appartenant à $R^3$, et le radical allyle ou méthallyle occupant la position 1 ou la position 6 du système bicyclique.

2. Imides de formule I selon la revendication 1 dans lesquels $R^1$ et $R^2$ représentent chacun l'hyrogène.

3. Imides de formule I selon la revendication 1 dans lesquels $R^3$ représente une liaison directe, un radical, $—C_rH_{2r}—$ dans lequel r désigne un nombre de 2 à 6, un radical $—CH_2CH_2{+}OCH_2CH_2]_m$ dans lequel m est égal à 2 ou, mieux, à 1, un radical du glycérol, un radical cycloaliphatique bivalent qui comporte un seul noyau et contient de 5 à 8 atomes de carbone, un radical aromatique bivalent en $C_6$—$C_{10}$ non substitué, ou un radical de formule II dans lequel T représente O, $SO_2$ ou, mieux, un méthylène ou un isopropylidène.

4. Imides de formule I selon la revendication 1 dans lesquels $R^3$ représente une liaison directe ou l'un des radicaux suivants:

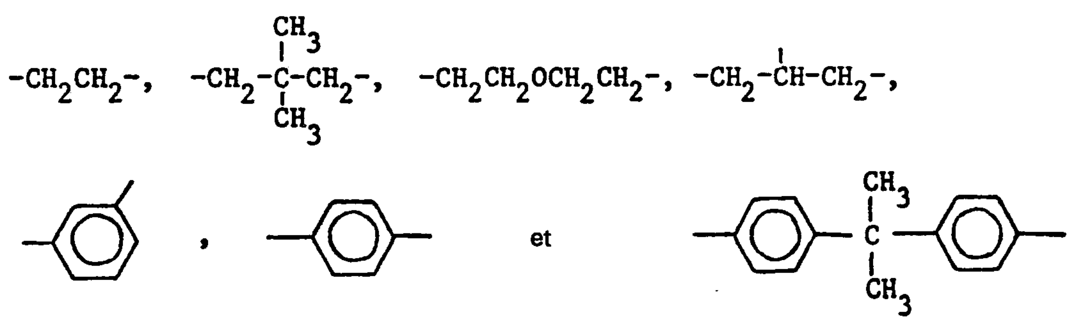

, , , , , et

5. Imides de formule I selon la revendication 1 dans lesquels $R^3$ représente une liaison directe, un radical éthylène ou un radical phénylène-1,4.

6. Procédé pour préparer des imides de formule I selon la revendication 1, procédé caractérisé en ce qu'on fait réagir un anhydride de formule III:

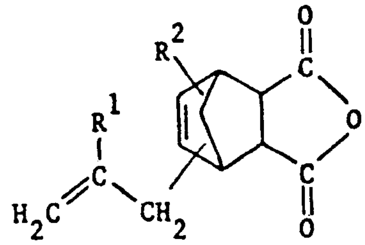

(III)

à température élevée et tout en chassant par distillation l'eau engendrée au cours de la réaction, avec un composé de formule IV:

$$H_2N—R^3(OH)_n \qquad (IV),$$

formules dans lesquelles $R^1$, $R^2$, $R^3$ et n ont les significations qui leur ont été données à la revendication 1.

7. Application des imides de formule I selon la revendication 1, en association avec une ou plusieurs résines époxydiques et, éventuellement, d'autres additifs usuels, à la fabrication de produits durcis.

**Claims**

1. An imide of the formula

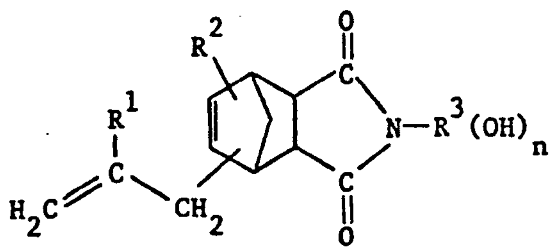

(I),

wherein $R^1$ and $R^2$ independently of one another are each hydrogen or methyl and n is 1, 2 or 3, and $R^3$ is a direct bond or a $C_2—C_{20}$ aliphatic radical, a mono- or polynuclear $G_5—C_{20}$ cycloaliphatic radical or a $C_6—C_{20}$ aromatic radical, a group of the formula II

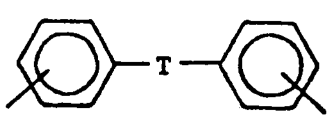

(II),

wherein T is methylene, isopropylidene, CO, O, S or $SO_2$, or is a $C_2—C_{20}$ aliphatic radical which has the formula $—CH_2CH_2—[OCH_2CH_2]_m$ or $—CH_2CH_2CH_2—[OCH_2CH_2CH_2]_m$ in which m is 1—10 and which is interrupted in the chain by O atoms, the OH groups being bound to different C atoms of $R^3$, and the allyl or methallyl group being bound in the 1- or 6-position of the bicyclic system.

2. An imide of the formula I according to claim 1 wherein $R^1$ and $R^2$ are each hydrogen.

3. An imide of the formula I according to claim 1 wherein $R^3$ is a direct bond, a group $—C_rH_{2r}—$ in which 4 is 2—6, a group $—CH_2CH_2—[OCH_2CH_2]_m$ in which m is 2 and especially 1, a radical of glycerol, a mononuclear, divalent, cycloaliphatic radical having 5—8 C atoms, an unsubstituted divalent $C_6—C_{10}$ aromatic radical, or a group of the formula II in which T is O, $SO_2$, and especially methylene or isopropylidene.

4. An imide of the formula I according to claim 1 wherein $R^3$ is a direct bond, or the radical

$-CH_2CH_2-$, 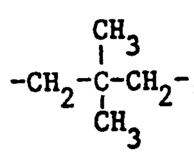, $-CH_2CH_2OCH_2CH_2-$, 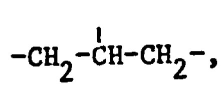,

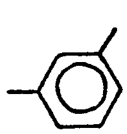, 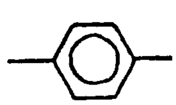 or 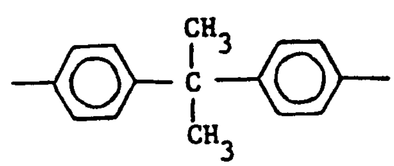

5. An imide of the formula I according to claim 1 wherein $R^3$ is a direct bond, ethylene or 1,4-phenylene.

6. A process for producing an imide of the formula I according to claim 1, which comprises reacting an anhydride of the formula III

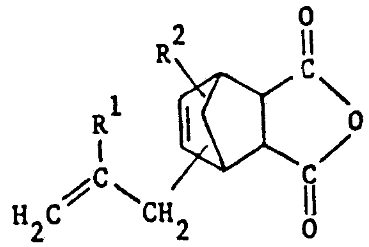

(III)

at elevated temperature and with removal by distillation of the water forming during the reaction, with a compound of the formula IV

$$H_2N—R^3(OH)_n \qquad (IV)$$

wherein $R^1$, $R^2$, $R^3$ and n have the meanings defined in claim 1.

7. Use of an imide of the formula I according to claim 1, together with one or more epoxy resins and optionally further customary additives, for producing cured products.